Europäisches Patentamt

European Patent Office

Office européen des brevets

(19)

(11) Publication number: **0 191 292 B1**

## (12) EUROPEAN PATENT SPECIFICATION

(45) Date of publication of patent specification: **12.06.91**   (51) Int. Cl.⁵: **A61K 7/021**

(21) Application number: **86100185.7**

(22) Date of filing: **08.01.86**

(54) **Cosmetics comprising a titanium oxide pigment.**

(30) Priority: **11.01.85 JP 3653/85**

(43) Date of publication of application:
**20.08.86 Bulletin 86/34**

(45) Publication of the grant of the patent:
**12.06.91 Bulletin 91/24**

(84) Designated Contracting States:
**BE CH DE FR GB IT LI**

(56) References cited:
**EP-A- 0 036 199
FR-A- 2 193 859
US-A- 3 422 185
US-A- 4 177 259
US-A- 4 309 411**

**CHEMICAL ABSTRACTS, vol. 98, no. 26, June 1983, page 365, abstract no. 221625h, Columbus, Ohio, US; & JP-A-58 49 307 (POLA CHEMICALS LTD) 23-03-1983**

**CHEMICAL ABSTRACTS, vol. 80, no. 1, 14th January 1974, Page 232, abstract no. 6785h, Columbus, Ohio, US; & JP-A-73 56 833 (NIHON KOKEN KOZYO CO. LTD) 09-08-1973**

**CHEMICAL ABSTRACTS, vol. 101, no. 14, Oc-**

tober 1984, page 342, abstract no. 116589g, Columbus, Ohio, US; & JP-A-59 98 009 (KANEBO LTD) 06-06-1984

(73) Proprietor: **SUMITOMO CHEMICAL COMPANY, LIMITED
Kitahama 4-chome 5-33
Chuo-ku Osaka 541(JP)**

(72) Inventor: **Saita, Kenji
10-5-102, Sonehigashinocho-2-chome
Toyonaka-shi(JP)**
Inventor: **Saegusa, Kunio
6-426, Ikkucho-2-chome
Niihama-shi(JP)**

(74) Representative: **Henkel, Feiler, Hänzel & Partner
Möhlstrasse 37
W-8000 München 80(DE)**

EP 0 191 292 B1

**Description**

BACKGROUND OF THE INVENTION
FIELD OF THE INVENTION

The present invention relates to makeup
a cosmetic extender consisting of a flaky titanium oxide of specific dimensions.

DESCRIPTION OF THE PRIOR ART

Makeup cosmetics are applied for the purpose of covering and coloring the skin appropriately and include, for example, powder foundations, oil foundations, pressed powders, lipsticks, and eyeshadows.

For makeup cosmetics, there are used various base materials for the purpose of providing the skin with moderate gloss and clarity feel, improving the applied cosmetics in spread and adherence, preventing them from the falling-off which may be caused by secretions such as sweat and sebum and bettering the cosmetics in the touch perceived on application and in processability for shaping them into final products. Jointly with the base materials, colorants are used to provide colors.

The various base materials include talc and other extenders, fats and oils, hydrocarbons, surfactants, etc.

A number of materials are known as the above extenders, but when used separately, none of the materials satisfies requirements for the extender to show appropriate gloss, spread, and adherence.

For example, talc, mica, kaolin, and sericite are known as naturally-occurring laminar clay minerals, but talc, mica, and sericite are inferior in adhesiveness. Kaolin, precipitated calcium carbonate, and the like are used to improve the adhesiveness but do not have spreadability at all. Accordingly, these minerals are used in combination for powder foundations, face powders, pressed powders, lipsticks, and rouges.

An additional problem of these minerals is that, when they are used jointly with other ingredients for cosmetics, such as oils, fats, and perfumes, these oils and fats may undergo rancidity or the perfumes may be deteriorated, on account of impurities, hydroxyl groups, or alkali metals contained in the minerals. For the purpose of overcoming this problem, there are proposed the method of compounding the clay mineral after dehydration thereof (Japanese Patent Application Laid-Open No. 16941/82) and the method of treating the mineral with a polyamino acid (Japanese Patent Application Laid-Open No. 145006/82). However, these methods are disadvantageous in that the complete removal of the impurities is impossible in spite of complicated operations required and the products, which need normally to be white, will be tinged with yellow-brown by the action of impurities (e.g. iron oxide) remaining in the treated minerals.

Furthermore, the clay minerals have the drawback of turning blackish on wetting with sweat, rain water, or the like, on account of their low refractive indexes.

There is also proposed a flaky pigment which is formed of 0.05 - 1 $\mu$m thick, 5 - 100 $\mu$m size platelet-shaped crystals of barium sulfate covered with thin layers of a metal oxide having a high refractive index (Japanese Patent Application Laid-Open No. 56833/73). However, this pigment also has drawbacks in that it requires a long production course comprising precipitation of barium sulfate and subsequent precipitation of the metal oxide and shines excessively as an extender, because the metal oxide layers are as thin as 0.01 to 0.1 $\mu$m and hence exhibit pearly gloss.

Further, there is known an attempt to grind mica-titania, which has been used primarily as a pearl pigment, thereby reducing the shine and using the ground product as an extender, but the spreadability thereof is insufficient.

For the colorant, on the other hand, there are used color pigments, inorganic pigments such as iron oxide, tar-derived colorants such as Red No. 226, and pearl pigments such as mica-titania exhibiting pearly or metallic luster.

Among these pigments, the pearl pigments need to be as thin as from 0.01 to 0.1 $\mu$m in order to meet the optical requirement therefor to develop pearly luster (Japanese Patent Publication No. 15579/60).

In addition, the pearl pigments should have a certain degree of size in order to reduce light scattering at the edges, for the purpose of achieving the intended optical effect. The acceptable sizes of mica-titania are 5 to 100 $\mu$m.

Such pearl pigments include crystals of guanine, basic lead carbonate, bismuth oxychloride, etc., and mica-titania that is formed of a mica coated with titanium oxide. While these pigments are in use for lipsticks, eyeshadows, nail enamels, etc. having brilliant, characteristic effect, guanine has difficulties in that it is expensive and its supply is limited since it is recovered from fish scales of a special kind, basic lead carbonate crystals and bismuth oxychloride crystals are undesirable for cosmetic use because of the

2

toxicity of lead or bismuth, and mica-titania has the difficulty of being chemically unstable under the influence of impurities contained in the mica and is still unsatisfactory in adhesiveness.

FR-A-2 193 859 discloses a pearl pigment obtained by covering small platelet-shaped anhydrite of 0.1 to 1 μm thickness and 5 to 70 μm in length with crystalline titanium dioxide of 5 to 500 nm in thickness.

## SUMMARY OF THE INVENTION

The present inventors found that flaky titanium oxide can be used as an extender, provided the flakes have an appropriate thickness and size and that makeup cosmetics comprising said flaky titanium oxide as an extender exhibit such excellent touch and standing stability in use that the prior art cosmetics could not attain. Based on this finding, the present invention has been accomplished.

Thus, the present invention involves a cosmetic makeup extender consisting of a flaky titanium oxide having a mean thickness of 0.1 to 3 μm and a mean size of 0.8 to 70 μm.

## DETAILED DESCRIPTION OF THE INVENTION

A group of flakes generally have distributions of dimensions. Accordingly, the size is specified as a mean size, that is, the mean of the value [(the largest diameter + the smallest diameter) of one flake/2] of 100 flakes, and the thickness also as the mean thickness of 100 flakes.

The flaky titanium oxide used as an extender and having a mean thickness of 0.1 to 3 μm and a mean size of 0.8 to 70 μm, exhibits moderate gloss, and is free of marked turbidity (hereinafter this type of titanium oxide is referred to as the low-gloss flaky titanium oxide).

The low-gloss flaky titanium oxide exhibits moderate gloss comparable to that of talc or sericite, spreadability comparable to that of talc, mica, or sericite and superior to that of kaolin, precipitated calcium carbonate, or conventional powdery titanium oxide, and adhesiveness superior to that of talc, mica, or sericite. Moreover, this type of flaky titanium oxide is chemically stable, scarcely deteriorates, has a high refractive index and hence undergoes no change in color on wetting with water.

Cosmetics comprising the low-gloss flaky titanium oxide are satisfactory in any of adhesiveness, spreadability, and moderate gloss and provide unprecedented smooth and moistlike feels and natural aesthetic finishes with suitable slight shine.

When the mean thickness of the low-gloss flaky titanium oxide is less than 0.1 μm, the flakes will exhibit high reflectivity and pearly luster and low mechanical strength, being liable to split.

The mean thickness of these titanium oxide flakes is at least 0.1 μm, preferably at least 0.2 μm.

As the mean thickness is increased over 0.1 μ, the metallic gloss of the flakes much decreases, and as the mean thickness is increased beyond 0.2 μm, the metallic gloss becomes far lower.

However, when the mean thickness exceeds 3 μm, the adherence and mellow feeling to the skin will be deteriorated.

Thus the mean thickness is desirably up to 2 μm, preferably up to 1 μm.

When the mean size of the low-gloss flaky titanium oxide is made less than 0.8 μm, the titanium oxide will become a too white pigment which has lost moderate gloss, feeling of clarity, and completely spreadability, though the adhesiveness is good.

As the mean size is increased, the spreadability becomes better. However, when the mean size exceed 70 μm, the particles of titanium oxide will tend to separate and hence become incapable of achieving the purpose of covering the skin uniformly. In consequence, the mean size is desirably up to 70 μm, preferably up to 40 μm.

Therefore, suitable low-gloss flaky titanium oxide has a mean thickness of 0.2 to 2 μm, particularly 0.2 to 1 μm, and a mean size of 0.8 to 70 μm, particularly 2 to 40 μm.

The flaky titanium oxide used in the present invention can be produced according to various processes.

Titanium oxide flakes of desired thickness can be obtained, for example, by a process (U.S. Patent No. 2,941,895) comprising applying a solution of titanium alkoxide in an organic solvent on a smooth flat surface, and cracking the resulting film into flakes by the action of steam, a process (Japanese Patent Publication No. 473/55) comprising applying a solution of titanium tetrachloride on a gelatin film, followed by dissolving the gelatin film, a process (Japanese Patent Publication No. 25280/64) comprising vacuum deposition, or a process (Japanese Patent Application Laid-Open No. 88121/83) comprising treating potassium titanate fibers with acid and subsequently with heat.

From the titanium oxide flakes obtained by the above-mentioned processes, those of a prescribed mean size then can be prepared by well-known methods ("Powder Engineering Handbook" edited by Iitani Koichi, published by Asakura Shoten Co., Ltd.), for example; a grinding method employing a dry ball mill, wet ball

3

mill, vibrating mill, roll mill, or jet mill and/or one or more methods selected from a vibrating screen method employing a gyro shifter or hammer screen, wet classification method employing a spiral classifier or hydraulic classifier, dry classification method employing a dynamic or centrifugal air classifier or the like, and ore floatation method.

The compounding proportions of the thus obtained flaky titanium oxide to form cosmetics are similar to those of conventional extenders and depend upon the nature of the cosmetic.

Of course, the titanium oxide may be used jointly with a conventional extender or pearl pigment, eg with a thinner flaky titanium oxide to be used as a pearl pigment and having a mean thickness of from 0.01 to below 0.1 μm, a mean size of 5 to 100 μm, high reflectivity, viz. high gloss, and good adhesiveness, which does not contain so much impurities as does mica, and is pure-white, free of marked turbidity, and chemically stable (hereinafter this type of flaky titanium oxide is referred to as the high-gloss flaky titanium oxide).

Cosmetics comprising besides the extenders according to the invention a high-gloss flaky titanium oxide of the above type are superior in spreadability and adhesiveness and produce clear coloring effect. In such cosmetics, the decomposition of the simultaneously blended organic compounds does not take place.

If the mean thickness of the high-gloss titanium oxide flakes is less than 0.01 μm, the mechanical strength thereof will be poor, and if the mean thickness is more than 0.1 μm, the reflectivity will be insufficient.

If the mean size of these flakes is less than 5 μm, the gloss of the product cosmetics will be too low, and if the mean size is larger than 100 μm, make-up effects including adherence of the product cosmetics will be deteriorated.

The low-gloss flaky titanium oxide is used as an extender, for instance, in proportions of 2 to 60% by weight for oil foundations and 20 to 90% by weight for pressed powders or powder foundations.

In general, if the compounding proportion is less than the above lower limit, the effect of the present invention will not be notable, and if the proportion is higher than the above upper limit, the moistlike feel and other comfortable feels of the product will be deteriorated since the product is composed substantially of titanium oxide flakes.

The low-gloss flaky titanium oxide may be used after transformation thereof into a colored extender by coating it with a colorant selected from; colored metal oxides, e.g. iron oxide, chromium oxide, and cobalt oxide; metal complex salts, e.g. ferrocyanides; colored metal hydroxides, e.g. iron hydroxide; organic dyes, e.g. FD & C Red No. 2 and FD & C Yellow No. 4; and organic pigments, e.g. aluminum lakes of the above dyes.

If used together with the low-gloss flaky titanium oxide extender the high-gloss flaky titanium oxide as a pearl pigment is used for instance, in proportions of 0.1 to 1% by weight for nail enamels, 0.1 to 1% by weight for lipsticks, and 2 to 80% by weight for eyeshadows.

Blending these two types of flaky titanium oxide to produce cosmetics can be carried out by known means such as a Henschel mixer, ribbon mixer, V-type blender, and the like.

The present invention is illustrated in more detail with reference to the following examples.

Light reflectivities (the higher reflectivity indicates that the gloss is the more metallic) of test samples were measured in the following way.

Measurement of reflectivity:

A pigment material was mixed by 10% by weight with nitrocellulose lacquer to obtain a test solution having the following components:

```
Pigment material                              10 parts (by wt.)

Nitrocellulose RS 1/4 (made by Daicel*        16    "

Isopropyl alcohol                              7    "

Isoamyl acetate                               35    "


* Chemical Industries, Ltd.)
```

| n-Butyl acetate | 29 parts (by wt.) |
|---|---|
| Diethylene glycol monobutyl ether | 3 " |

These mateirals were thoroughly mixed to disperse the powder. The dispersion was spread with a doctor blade to a thickness of 75 $\mu$m on a white-black hiding-power testing chart fixed on a surface plate, and was solidified to form a film.

Then the surface gloss of the film was measured on the film portion lying on a black area of the chart in accordance with the specular gloss measuring method of JIS-Z8741 (Japanese Industry Standard) at a light incident angle of 20 degree and a reflection angle of 20 degree.

The found surface gloss was regarded as the reflectivity of the sample.

(Sericite and talc which are conventional extenders have reflectivities of 5 to 30%, and pearl pigments, including various grades, have reflectivities of 40% at the lowest and 60% at the highest.)

Reflectivities of different grades of flaky titanium oxide according to the present invention are shown in Table 1 and reflectivities of conventional pigments are shown in Table 2. (The size and thickness of particles were measured with a scanning electron microscope.)

Example 1 and Comparative Examples 1 to 4

A powder foundation having a composition as shown in Table 3 was prepared by using a low-gloss flaky titanium oxide of Sample No. 1 shown in Table 1.

For comparison, other powder foundations were prepared by using a talc, kaolin, synthetic polymer powder, and mica-titania (MP 1005) in place of the low-gloss flaky titanium oxide of Sample No. 1.

The spread, adherence, smooth feel, gloss, and color tone of the foundations were evaluated by sensory test employing 20 women as panelists, where the evaluation results were ranked into 5 groups with the best results rated as 5 points. Results of the evaluation are shown in Table 3.

It can be seen from Table 3 that the powder foundation including the low-gloss flaky titanium oxide extender is superior to the talc-based one in adherence and smooth feel, to the kaolin-based one in spread and gloss, to the synthetic polymer powder-based one in adherence and smooth feel, and to the mica-titania-based one in gloss.

Example 2 and Comparative Example 5

An oil foundation was prepared by using a low-gloss flaky titanium oxide extender of Sample No. 2 shown in Table 1.

For comparison, an oil foundation was prepared by using a conventional powdery titanium oxide. Results of evaluating these foundations are shown in Table 4.

Example 3 and Comparative Example 6

A pressed powder having a composition as shown in Table 5 was prepared by using a low-gloss flaky titanium extender oxide of No. 2 shown in Table 1.

For comparison another pressed powder was prepared by using a talc. Results of evaluating both the pressed powders are shown in Table 5.

The pressed powder including the low-gloss flaky titanium oxide extender, as compared with the talc-based pressed powder, was superior in adherence and exhibited moderated gloss though comparable in spread.

Example 4 and Comparative Example 7

A lipstick was prepared by using a low-gloss flaky titanium oxide extender of Sample No. 3 shown in Table 1.

For comparison, another lipstick was prepared by using a conventional powdery titanium oxide. Results of evaluating both the lipsticks are shown in Table 6.

The lipstick including the low-gloss flaky titanium oxide extender, as compared with the one based on the conventional powdery titanium oxide, was superior in spread and gloss and showed a vivid color, though

comparable in adherence.

Table 1　Size and reflectivity of flaky titanium oxide extenders

| Sample No. | Size | | | | Mean size (μm) | Mean thickness (μm) | Reflectivity (%) | Goodness |
|---|---|---|---|---|---|---|---|---|
| | 14 μm> | 14 μm - 40 μm | 40 μm - 70 μm | >70 μm | | | | |
| 1 | 60% | 26% | 9% | 5% | 4 | 0.3 | 7.1±0.2 | |
| 2 | 25 | 51 | 19 | 5 | 18 | 0.5 | 15.5±0.3 | |
| 3 | 0 | 55 | 26 | 19 | 50 | 0.7 | 29.8±0.3 | |

6

Table 2   Reflectivity of pigment

| Pigment | Shape | Reflectivity | Use |
|---|---|---|---|
| DM-OA mica (made by Topy Industries, Ltd.) | Flake | 23.2±0.3 | |
| Sericite FSE (made by Kokonen Company, Ltd.) | " | 13.4±0.2 | |
| Fine powder of talc | " | 5.1±0.2 | Ex-tender |
| Powdery titanium oxide (A-100, supplied by Ishihara Sangyo Co., Ltd.) | Powder | 22.1±0.3 | |
| Pigment (mica-titania, MP-47, supplied by Merck Co.) | Flake | 60.7±1.1 | Pearl pigment |
| Pigment (mica-titania MP-1005, supplied by Merck Co.) | " | 42.7±0.8 | |

EP 0 191 292 B1

Table 3   Powder foundation

| Composition (weight %) | Example 1 | Comparative Example 1 | Comparative Example 2 | Comparative Example 3 | Comparative Example 4 |
|---|---|---|---|---|---|
| Red iron oxide | 2.0 | 2.0 | 2.0 | 2.0 | 2.0 |
| Yellow iron oxide | 6.0 | 6.0 | 6.0 | 6.0 | 6.0 |
| Black iron oxide | 0.8 | 0.8 | 0.8 | 0.8 | 0.8 |
| Powdery titanium oxide (A-100) | 8.0 | 8.0 | 8.0 | 8.0 | 8.0 |
| Kaolin | - | - | 50.0 | - | - |
| Sericite | 24.9 | 24.9 | 24.9 | 24.9 | 24.9 |
| Synthetic polymer powder (nylon) | - | - | - | 50 | - |
| Talc | - | 50 | - | - | - |
| Mica-titania | - | - | - | - | 50.0 |
| Low-gloss flaky titanium oxide | 50.0 | - | - | - | - |
| Squalane | 2.0 | 2.0 | 2.0 | 2.0 | 2.0 |
| Liquid paraffin | 2.0 | 2.0 | 2.0 | 2.0 | 2.0 |
| Octyldecyl myristate | 2.0 | 2.0 | 2.0 | 2.0 | 2.0 |

- cont'd -

Table 3 (cont'd)

| | | | | | | |
|---|---|---|---|---|---|---|
| White vaseline | | 2.0 | 2.0 | 2.0 | 2.0 | 2.0 |
| Preservative | | 0.1 | 0.1 | 0.1 | 0.1 | 0.1 |
| Perfume | | 0.2 | 0.2 | 0.2 | 0.2 | 0.2 |
| Evalua-tion | Spread | 4.5 | 4.5 | 2.0 | 5.0 | 4.5 |
| | Adherence | 5.0 | 2.0 | 5.0 | 1.0 | 4.5 |
| | Gloss | 5.0 | 4.0 | 3.0 | 3.0 | 1.0 |
| | Smooth and moistlike feel | 5.0 | 2.0 | 4.0 | 3.0 | 5.0 |

EP 0 191 292 B1

Table 4  Oil foundation

| Composition (weight %) | Example 2 | Comparative Example 5 |
|---|---|---|
| Red iron oxide | 2.0 | 2.0 |
| Yellow iron oxide | 4.0 | 4.0 |
| Black iron oxide | 1.0 | 1.0 |
| Powdery titanium oxide (A-100) | - | 35.0 |
| Kaolin | 8.0 | 8.0 |
| Low-gloss flaky titanium oxide | 35.0 | - |
| Squalane | 10.0 | 10.0 |
| Microcrystalline wax | 6.0 | 6.0 |
| Ozocerite | 10.0 | 10.0 |
| White vaseline | 5.0 | 5.0 |
| Liquid paraffin | 17.8 | 17.8 |
| Sorbitan monooleate | 1.0 | 1.0 |
| Antioxidant | 0.1 | 0.1 |
| Preservative | 0.1 | 0.1 |
| Perfume | 0.2 | 0.2 |
| Evalua-tion | Spread | 5.0 | 2.0 |
| | Adherence | 5.0 | 5.0 |
| | Gloss | 5.0 | 2.0 |
| | Smooth and moistlike feels | 5.0 | 2.0 |

Table 5 Pressed powder

| Composition (weight %) | | Example 3 | Comparative Example 6 |
|---|---|---|---|
| Red iron oxide | | 0.4 | 0.4 |
| Yellow iron oxide | | 0.2 | 0.2 |
| Black iron oxide | | 0.1 | 0.1 |
| Sericite | | 22.1 | 22.1 |
| Talc | | - | 70.0 |
| Low-gloss flaky titanium oxide | | 70.0 | - |
| Zinc stearate | | 3.0 | 3.0 |
| Squalane | | 2.0 | 2.0 |
| Methylphenylpolysiloxane | | 2.0 | 2.0 |
| Antioxidant | | Slight amount | Slight amount |
| Preservative | | Slight amount | Slight amount |
| Perfume | | 0.2 | 0.2 |
| Evalua-tion | Spread | 4.8 | 4.8 |
| | Adherence | 5.0 | 3.5 |
| | Gloss | 4.5 | 4.0 |
| | Smooth and moistlike feels | 4.5 | 3.8 |

Table 6  Lipstick

| Composition (weight %) | Example 4 | Comparative Example 7 |
|---|---|---|
| Castor oil | 45.3 | 45.3 |
| Hexadecyl alcohol | 25.0 | 25.0 |
| Lanolin | 4.0 | 4.0 |
| Beeswax | 5.0 | 5.0 |
| Ozocerite | 4.0 | 4.0 |
| Candelilla wax | 7.0 | 7.0 |
| Carnauba wax | 2.0 | 2.0 |
| Antioxidant | Slight amount | Slight amount |
| Powdery titanium oxide (A-100) supplied by Ishihara Sangyo Co., Ltd. | - | 2.0 |
| Low-gloss flaky titanium oxide | 2.0 | - |
| D & C Red No. 7 | 0.5 | 0.5 |
| D & C Red No. 9 | 2.5 | 2.5 |
| D & C Red No. 33 Al lake | 2.5 | 2.5 |
| D & C Orange No. 5 | 0.2 | 0.2 |
| Perfume | Slight amount | Slight amount |

| Evaluation | | Example 4 | Comparative Example 7 |
|---|---|---|---|
| | Spread | 5.0 | 4.0 |
| | Adherence | 4.2 | 4.2 |
| | Gloss | 5.0 | 3.0 |
| | Color tone | 4.7 | 3.8 |

**Claims**

1. Cosmetic make up extender consisting of a flaky titanium oxide having a mean thickness of from 0.1 to 3 µm and a mean size of 0.8 to 70 µm.

**Revendications**

1. Charge pour produit de maquillage cosmétique consistant en un oxyde de titane en paillettes ayant une épaisseur moyenne de 0,1 à 3 µm et une dimension moyenne de 0,8 à 70 µm.

**Ansprüche**

1. Streckmittel für ein kosmetisches Make-up, bestehend aus flockenförmigem Titanoxid einer mittleren Dicke von 0,1 bis 3 µm und einer mittleren Größe von 0,8 bis 70 µm.